# EUROPÄISCHE PATENTSCHRIFT

(11) **EP 1 699 776 B1**
(45) Veröffentlichungstag und Bekanntmachung des Hinweises auf die Patenterteilung: **12.11.2008**
(21) Anmeldenummer: 04804133.9
(22) Anmeldetag: 21.12.2004
(51) Int. Cl.: C07D 323/06

(54) **VERFAHREN ZUR ABTRENNUNG VON TRIOXAN AUS EINEM TRIOXAN/FORMALDEHYD/WASSER-GEMISCH MITTELS DRUCKWECHSEL-REKTIFIKATION**
METHOD FOR SEPARATING TRIOXANE FROM A TRIOXANE/FORMALDEHYDE/WATER MIXTURE BY MEANS OF PRESSURE CHANGE RECTIFICATION
PROCEDE DE SEPARATION DE TRIOXANE D'UN MELANGE TRIOXANE/FORMALDEHYDE/EAU A L'AIDE DE LA RECTIFICATION PAR MODIFICATION DE PRESSION

(30) Priorität: 23.12.2003 DE 10361516
(43) Veröffentlichungstag der Anmeldung: 13.09.2006
(73) Patentinhaber: BASF SE, 67056 Ludwigshafen (DE)
(72) Erfinder: SIEGERT, Markus, 69126 Heidelberg (DE); LANG, Neven, 68163 Mannheim (DE); STROEFER, Eckhard, 68163 Mannheim (DE); STAMMER, Achim, 67251 Freinsheim (DE); FRIESE, Thorsten, 68163 Mannheim (DE); HASSE, Hans, 67661 Kaiserslautern (DE)
(74) Vertreter: Isenbruck, Günter
(86) Internationale Anmeldenummer: PCT/EP2004/014535
(87) Internationale Veröffentlichungsnummer: WO 2005/063733

(56) Entgegenhaltungen:
- EP-A- 0 583 907
- EP-A- 0 692 481
- WO-A-99/05137
- WO-A-03/097630
- DE-A- 1 543 340
- DE-A- 1 668 867
- DE-A- 3 621 722
- US-A- 4 110 298
- US-A- 4 332 644
- US-A- 5 523 419
- US-B1- 6 201 136

## Beschreibung

Die Erfindung betrifft ein Verfahren zur Abtrennung von Trioxan aus einem Trioxan/Formaldehyd/Wasser-Gemisch sowie ein Verfahren zur Herstellung von Trioxan.

Trioxan wird in der Regel durch Destillation von wässriger Formaldehydlösung in Gegenwart saurer Katalysatoren hergestellt. Dem Formaldehyd und Wasser enthaltenden Destillat wird anschließend das Trioxan durch Extraktion mit halogenierten Kohlenwasserstoffen, wie Methylenchlorid oder 1,2-Dichlorethan, oder anderen, mit Wasser nicht mischbaren Lösungsmitteln entzogen.

DE-A 1 668 867 beschreibt ein Verfahren zur Abtrennung von Trioxan aus Wasser, Formaldehyd und Trioxan enthaltenen Gemischen durch Extraktion mit einem organischen Lösungsmittel. Dabei wird eine aus zwei Teilstrecken bestehende Extraktionsstrecke an einem Ende mit einem üblichen organischen, mit Wasser praktisch nicht mischbaren Extraktionsmittel für Trioxan beschickt, am anderen Ende mit Wasser. Zwischen den beiden Teilstrecken wird das zu trennende Destillat der Trioxan-Synthese zuführt. Auf der Seite der Lösungsmittelzuführung wird dann eine wässrige Formaldehydlösung und auf der Seite der Wasserzuführung eine praktisch formaldehydfreie Lösung von Trioxan in dem Lösungsmittel erhalten. In einem Beispiel wird das bei der Trioxan-Synthese entstandene Destillat aus 40 Gew.-% Wasser, 35 Gew.-% Trioxan und 25 Gew.-% Formaldehyd in den Mittelteil einer Pulsationskolonne eindosiert, am oberen Kolonnenende Methylenchlorid und am unteren Kolonnenende Wasser zugeführt. Dabei wird am unteren Kolonnenende eine etwa 25 gew.-%ige Lösung von Trioxan in Methylenchlorid und am oberen Kolonnenende eine etwa 30 gew.-%ige wässrige Formaldehydlösung erhalten.

Nachteil dieser Verfahrensweise ist der Anfall an Extraktionsmittel, welches aufgereinigt werden muss. Bei den verwendeten Extraktionsmitteln handelt es sich zum Teil um Gefahrenstoffe (T oder T⁺-Stoffe im Sinne der deutschen Gefahrenstoffverordnung), deren Handhabung besondere Vorsichtsmaßnahmen erfordert.

DE-A 197 32 291 beschreibt ein Verfahren zur Abtrennung von Trioxan aus einem wässrigen Gemisch, das im Wesentlichen aus Trioxan, Wasser und Formaldehyd besteht, bei dem man dem Gemisch Trioxan durch Pervaporation entzieht und das an Trioxan angereicherte Permeat durch Rektifikation in Trioxan und ein azeotropes Gemisch aus Trioxan, Wasser und Formaldehyd trennt. In dem Beispiel wird ein wässriges Gemisch bestehend aus 40 Gew.-% Trioxan, 40 Gew.-% Wasser und 20 Gew.-% Formaldehyd in einer ersten Destillationskolonne unter Normaldruck in ein Wasser/Formaldehyd-Gemisch und in ein azeotropes Trioxan/Wasser/Formaldehyd-Gemisch getrennt. Das azeotrope Gemisch wird in eine Pervaporationseinheit geleitet, welche eine Membran aus Polydimethylsiloxan mit einem hydrophoben Zeolithen enthält. Das mit Trioxan angereicherte Gemisch wird in einer zweiten Destillationskolonne unter Normaldruck in Trioxan und wiederum in ein azeotropes Gemisch aus Trioxan, Wasser und Formaldehyd aufgetrennt. Dieses azeotrope Gemisch wird vor die Pervaporationsstufe zurückgeführt.

Nachteilig an dieser Verfahrensweise sind die sehr hohen Investitionen für die Pervaporationseinheit.

Aufgabe der Erfindung ist es, ein Verfahren zur Abtrennung von Trioxan aus azeotropen Trioxan/Formaldehyd/Wasser-Gemischen bereitzustellen, welches ohne die Extraktionsschritte oder Pervaporationsschritte des Standes der Technik auskommt.

Gelöst wird diese Aufgabe durch ein Verfahren zur Abtrennung von Trioxan aus einem Einsatzstrom I aus Formaldehyd, Trioxan und Wasser, bei dem
a) ein Einsatzstrom I, der als Hauptkomponente Formaldehyd und als Nebenkomponenten Trioxan und Wasser enthält, bereitgestellt wird,
b) der Einsatzstrom I mit einem Rückführstrom VII, der als Hauptkomponente Trioxan und als Nebenkomponenten Formaldehyd und Wasser enthält, gemischt wird, wobei ein Einsatzstrom Ia, der als Hauptkomponente Formaldehyd und als Nebenkomponenten Trioxan und Wasser enthält, erhalten wird,
c) der Einsatzstrom Ia in einer ersten Destillationsstufe bei einem Druck von 0,1 bis 2,5 bar destilliert wird, wobei ein Strom II, der als Hauptkomponente Formaldehyd und als Nebenkomponente Wasser enthält, und ein Strom III, der als Hauptkomponente Trioxan und als Nebenkomponenten Wasser und Formaldehyd enthält, erhalten werden,
d) der Strom III, gegebenenfalls nach Abtrennung von Leichtsiedern aus dem Strom III in einer Leichtsieder-Abtrennstufe, in einer zweiten Destillationsstufe bei einem Druck von 0,2 bis 17,5 bar destilliert wird, wobei der Druck in der zweiten Destillationsstufe um 0,1 bis 15 bar höher als der Druck in der ersten Destillationsstufe ist, wobei ein Strom IV, der im wesentlichen aus Trioxan besteht, und ein Strom V, der als Hauptkomponente Trioxan und als Nebenkomponenten Wasser und Formaldehyd enthält, erhalten wird,
e) gegebenenfalls der Strom V mit einem Strom IX, der als Hauptkomponente Wasser enthält, gemischt wird, wobei ein Strom Va mit höherem Wassergehalt als Strom V, wobei der Strom Va als Hauptkomponenten Trioxan und als Nebenkomponenten Wasser und Formaldehyd enthält, erhalten wird,
f) der Strom V bzw. Va in einer dritten Destillationsstufe bei einem Druck von 1 bis 10 bar destilliert wird, wobei ein Strom VI, der im wesentlichen aus Wasser besteht, und der Rückführstrom VII, der als Hauptkomponente Trioxan und als Nebenkomponenten Wasser und Formaldehyd enthält, erhalten werden.

Die Hauptkomponente ist die Komponente mit dem größeren bzw. größten Massenanteil an dem betreffenden Gemisch. Vorzugsweise beträgt der Massenanteil der Hauptkomponente an dem jeweiligen Gemisch mindestens 50 Gew.-%.

Es ist bekannt, dass Trioxan, Formaldehyd und Wasser ein ternäres Azeotrop bilden, welches bei einem Druck von 1 bar die Zusammensetzung 69,5 Gew.-% Trioxan, 5,4 Gew.-% Formaldehyd und 25,1 Gew.-% Wasser aufweist.

Erfindungsgemäß wird dieses Azeotrop durch Druckwechseldestillation umgangen, bei dem eine erste und eine zweite Destillation bei verschiedenen Drücken durchgeführt werden. In einer ersten Destillationskolonne, welche bei niedrigerem Druck betrieben wird, wird das Ausgangsgemisch Ia in ein Trioxan/Wasser-Gemisch mit geringem Formaldehyd-Gehalt III und ein im Wesentlichen trioxanfreies Formaldehyd/Wasser-Gemisch II aufgetrennt. Das Formaldehyd/Wasser-Gemisch II kann in die Trioxan-Synthese zurückgeführt werden. In einer zweiten, bei höherem Druck betriebenen Destillationskolonne wird das erhaltene Trioxan/Formaldehyd/Wasser-Gemisch III in reines Trioxan und ein Trioxan/Formaldehyd/Wasser-Gemisch V mit niedrigerem Trioxan-Gehalt aufgetrennt. Erfindungsgemäß wird weiterhin das Trioxan/Formaldehyd/Wasser-Gemisch V (bzw. Va) in einer dritten Destillationskolonne in im Wesentlichen reines Wasser VI und ein Trioxan/Formaldehyd/Wasser-Gemisch mit höherem Trioxan-Gehalt VII aufgetrennt. Dieses wird vor die erste Destillationskolonne zurückgeführt. Vorzugsweise wird der Wassergehalt des Gemischs V vor der Abtrennung von Wasser in der dritten Destillationskolonne durch Zumischung eines wasserhaltigen Stroms IX erhöht.

Als Destillationskolonnen sind beliebige Destillationskolonnen wie Packungs- und Bodenkolonnen geeignet. Diese können beliebige Einbauten, Packungen oder Füllkörperschüttungen enthalten.

Der Druck in der zweiten Destillationsstufe ist um 0,1 bis 15 bar höher als der Druck in der ersten Destillationsstufe. Vorzugsweise beträgt diese Druckdifferenz 1,0 bis 10 bar, besonders bevorzugt 1,5 bis 5 bar.

Alle Druckangaben beziehen sich auf den Druck am Kopf der jeweiligen Kolonne.

Die erste Destillationsstufe wird bei einem Druck von 0,1 bis 2,5 bar, vorzugsweise 0,5 bis 2,0 bar durchgeführt. Die erste Destillationsstufe wird im Allgemeinen in einer Destillationskolonne mit mindestens 2, vorzugsweise 2 bis 50, besonders bevorzugt 4 bis 25 theoretischen Stufen durchgeführt. Im Allgemeinen umfasst der Abtriebsteil dieser Kolonne mindestens 25 %, vorzugsweise 50 bis 90 % der theoretischen Stufen dieser Kolonne.

Der Einspeisungsstrom Ia enthält im Allgemeinen 55 bis 85 Gew.-% Formaldehyd, 15 bis 35 Gew.-% Wasser und 1,0 bis 30 Gew.-% Trioxan. Dieser Strom Ia wird in einen Strom II, der vorzugsweise am Kolonnenkopf abgezogen wird, und ein Strom III, der vorzugsweise am Kolonnensumpf abgezogen wird, aufgetrennt.

Der Strom II enthält im Allgemeinen weniger als 1 Gew.-%, bevorzugt weniger als 0,1 Gew.-% Trioxan, besonders bevorzugt weniger als 0,01 Gew.-% Trioxan. Beispielsweise setzt sich der Strom II wie folgt zusammen: 65 bis 85 Gew.-% Formaldehyd, 15 bis 35 Gew.-% Wasser und 0 bis 1 Gew.-% Trioxan. Der Strom III enthält im Allgemeinen mehr als 50 Gew.-%, vorzugsweise mehr als 60 Gew.-%, besonders bevorzugt mehr als 70 Gew.-% Trioxan. Beispielsweise setzt sich der Strom III wie folgt zusammen: 3 bis 20 Gew.-% Formaldehyd, 10 bis 30 Gew.-% Wasser und 60 bis 80 Gew.-% Trioxan.

Der Strom II wird vorzugsweise in die Trioxan-Synthese zurückgeführt.

Die Ströme Ia, III, V, Va und VII können noch bis zu 15 Gew.-% Leichtsieder enthalten. Übliche Leichtsieder, die bei der Trioxan-Synthese und der nachfolgenden destillativen Trennung gebildet werden können, sind Methylformiat, Methylal, Dimethoxydimethylether, Trimethoxydimethylether, Methanol, Ameisensäure sowie weitere Halb- und Vollacetale. Zur Abtrennung dieser Leichtsieder kann optional zwischen der ersten und der zweiten Destillationsstufe eine Leichtsieder-Abtrennstufe durchgeführt werden. Dabei werden die Leichtsieder vorzugsweise über den Kopf einer Leichtsieder-Abtrennkolonne, welche im Allgemeinen bei einem Druck von 0,1 bis 5 bar, vorzugsweise bei einem Druck von 1,0 bis 2,5 bar betrieben wird, abgetrennt. Im Allgemeinen weist die Leichtsieder-Abtrennkolonne mindestens 2 theoretische Stufen, vorzugsweise 15 bis 50 theoretische Stufen auf. Im Allgemeinen umfasst der Abtriebsteil dieser Kolonne 25 bis 90 %, vorzugsweise 50 bis 75 % der theoretischen Stufen dieser Kolonne. Der Gehalt der gegenüber Trioxan leichter siedenden Komponenten im Sumpfaustrag der Leichtsieder-Abtrennkolonne beträgt im Allgemeinen weniger als 5 Gew.-%, bevorzugt weniger als 2,5 Gew.-%, besonders bevorzugt weniger als 1,5 Gew.-%.

Im Allgemeinen wird eine Leichtsieder-Abtrennung durchgeführt.

Der Strom III wird in einer zweiten Destillationsstufe bei einem Druck von 0,2 bis 17,5 bar in einen Strom IV aus im Wesentlichen reinem Trioxan und einen Strom V, der als Hauptkomponente Trioxan und daneben Wasser und Formaldehyd enthält, aufgetrennt. Diese zweite Destillationsstufe wird vorzugsweise bei 2,5 bis 10 bar durchgeführt. Im Allgemeinen wird diese zweite Destillationsstufe in einer Destillationskolonne mit mindestens 2 theoretischen Böden, vorzugsweise 10 bis 50 theoretischen Böden, durchgeführt, wobei der Strom IV als Sumpfabzugsstrom oder als Seitenabzugsstrom im Abtriebsteil der Kolonne anfällt und der Strom V als Kopfabzugsstrom anfällt. Im Allgemeinen umfasst der Abtriebsteil dieser Destillationskolonne 25 bis 90 %, vorzugsweise 50 bis 75 % der theoretischen Stufen dieser Kolonne.

Im Allgemeinen enthält der Strom IV 95 bis 100 Gew.-%, vorzugsweise 99 bis 100 Gew.-% Trioxan und 0 bis 5 Gew.-%, vorzugsweise 0 bis 1 Gew.-% Wasser und Nebenkomponenten. Nebenkomponenten sind insbesondere die oben genannten Leichtsieder, aber auch höher als Trioxan siedende Komponenten. Besonders bevorzugt ist der Gehalt an Wasser und Nebenkomponenten im Trioxan-Strom IV < 0,1 %. Er kann sogar < 0,01 % sein. Der Strom V enthält beispielsweise 5 bis 20 Gew.-% Formaldehyd, 15 bis 35 Gew.-% Wasser und 50 bis 75 Gew.-% Trioxan.

In einer bevorzugten Ausführungsform des erfindungsgemäßen Verfahrens wird dem Strom V vor Durchführung des dritten Destillationsschrittes ein wasserhaltiger Strom IX zugemischt, wobei ein Strom Va resultiert, der einen höheren Wassergehalt als der Strom V aufweist. Im Allgemeinen enthält der Strom Va 25 bis 100 Gew.-% Wasser. Beispielsweise enthält der Strom Va 5 bis 20 Gew.-% Formaldehyd, 25 bis 45 Gew.-% Wasser und 40 bis 65 Gew.-% Trioxan.

Der Strom V bzw. Va wird in einer dritten Destillationsstufe bei einem Druck von 1 bis 10 bar in einen Strom VI, der im Wesentlichen aus Wasser besteht, und einen Rückführstrom VII, der als Hauptkomponente Trioxan und daneben Wasser und Formaldehyd enthält, aufgetrennt. Vorzugsweise wird die dritte Destillationsstufe bei einem Druck von 2,5 bis 5 durchgeführt. Im Allgemeinen wird die dritte Destillationsstufe in einer Destillationskolonne mit mindestens 2 theoretischen Böden, vorzugsweise 10 bis 50 theoretischen Böden, durchgeführt, wobei der Wasserstrom VI als Sumpfabzugsstrom oder als Seitenabzugsstrom im Abtriebsteil der Kolonne und der Rückführstrom VII als Kopfabzugsstrom erhalten werden. Der Abtriebsteil dieser Kolonne umfasst im Allgemeinen 25 bis 95 %, vorzugsweise 70 bis 90 % der theoretischen Stufen dieser Kolonne.

Eine bevorzugte Destillationskolonne für die dritte Destillationsstufe ist eine Trennwandkolonne, wie sie beispielsweise in US 2,471,134, US 4,230,533, EP-A 0 122 367, EP-A 0 126 288 und EP-A 0 133 510 beschrieben ist.

Der Wasserstrom VI besteht vorzugsweise zu mehr als 95 Gew.-%, besonders bevorzugt zu mehr als 99 Gew.-% aus Wasser. Beispielsweise enthält der Strom VI 99 bis 100 Gew.-% Wasser und 0 bis 1 Gew.-% Formaldehyd.

Der Strom VII enthält beispielsweise 5 bis 40 Gew.-% Formaldehyd, 5 bis 40 Gew.-% Wasser und 50 bis 80 Gew.-% Trioxan.

Der Strom VII kann teilweise oder vollständig vor die erste Destillationsstufe zurückgeführt werden, vorzugsweise wird er im Wesentlichen vollständig vor die erste Destillationsstufe zurückgeführt. Dabei wird er mit dem Einsatzstrom I gemischt.

Gegenstand der vorliegenden Erfindung ist auch ein Verfahren zur Herstellung von Trioxan aus einer wässrigen Formaldehydlösung, bei dem der Formaldehyd, Trioxan und Wasser enthaltende Einsatzstrom I in einer vorgelagerten Trioxan-Synthesestufe aus einer wässrigen Formaldehydlösung hergestellt wird und anschließend aus dem Strom I wie vorstehend beschrieben Trioxan abgetrennt wird.

Gegenstand der vorliegenden Erfindung ist auch ein Verfahren zur Herstellung von Trioxan aus einer wässrigen Formaldehydlösung, bei dem der Formaldehyd, Trioxan und Wasser enthaltende Einsatzstrom I in einer vorgelagerten Trioxan-Synthesestufe aus einer wässrigen Formaldehydlösung hergestellt wird und anschließend aus dem Strom I wie vorstehend beschrieben Trioxan abgetrennt wird. Alternativ dazu können die Trioxansynthese und die erste Destillationsstufe in einer Reaktivdestillation vereinigt werden.

In einer Ausführungsform des erfindungsgemäßen Verfahrens wird ein Strom X aus einer wässrigen Formaldehydlösung einer vorgelagerten Trioxan-Synthesestufe zugeführt und in Gegenwart saurer homogen oder heterogen vorliegender Katalysatoren wie Ionenaustauscherharze, Zeolithe, Schwefelsäure und p-Toluolsulfonsäure bei einer Temperatur von im Allgemeinen 70 bis 130 °C umgesetzt. Dabei kann in einer Destillationskolonne oder einem Verdampfer (Reaktivverdampfer) gearbeitet werden. Das Produktgemisch aus Trioxan/Formaldehyd und Wasser fällt dann als dampfförmiger Brüdenabzugsstrom des Verdampfers bzw. als Kopfabzugsstrom am Kopf der Kolonne an. Die Trioxan-Synthesestufe kann auch in einem Festbett- oder Fließbettreaktor an einem heterogenen Katalysator, z. B. einem Ionenaustauscherharz oder Zeolith, durchgeführt werden.

In einer weiteren Ausführungsform des erfindungsgemäßen Verfahrens werden die Trioxan-Synthesestufe und die erste Destillationsstufe als Reaktivdestillation in einer Reaktionskolonne durchgeführt. Diese kann im Abtriebsteil ein Katalysator-Festbett aus einem heterogenen sauren Katalysator enthalten. Alternativ kann die Reaktivdestillation auch in Gegenwart eines homogenen Katalysators durchgeführt werden, wobei der saure Katalysator zusammen mit der wässrigen Formaldehyd-Lösung im Kolonnensumpf vorliegt.

Im Allgemeinen enthält die wässrige Formaldehydlösung, die der Trioxan-Synthesestufe zugeführt wird, 60 bis 85 Gew.-% Formaldehyd und 15 bis 40 Gew.-% Wasser. Diese Lösung kann in einem vorgelagerten Aufkonzentrierungsschritt aus einer wässrigen Formaldehydlösung mit niedrigerer Formaldehyd-Konzentration erhalten werden. Der Aufkonzentrierungsschritt kann beispielsweise in einem Verdampfer, vorzugsweise einem Fallfilmverdampfer, durchgeführt werden.

Der vorgelagerte Aufkonzentrierungsschritt kann beispielsweise wie in DE-A 199 25 870 beschrieben durchgeführt werden.

In einer Ausführungsform des erfindungsgemäßen Verfahrens wird ein Strom XI einer wässrigen Formaldehydlösung in einem Verdampfer, vorzugsweise einem Fallfilmverdampfer, aufkonzentriert, wobei ein Strom X aus einer wässrigen Formaldehydlösung mit höherer Formaldehyd-Konzentration erhalten wird. Der Brüdenabzugsstrom des Verdampfers, welcher stark an Formaldehyd abgereichert ist, wird als wasserhaltiger Strom IX mit dem Strom V gemischt. Strom XI enthält beispielsweise 50 bis 70 Gew.-% Formaldehyd und 30 bis 50 Gew.-% Wasser. Strom X enthält beispielsweise 65 bis 80 Gew.-% Formaldehyd und 20 bis 35 Gew.-% Wasser. Strom IX enthält beispielsweise 10 bis 25 Gew.-% Formaldehyd und 75 bis 90 Gew.-% Wasser.

Das erhaltene Rein-Trioxan, dessen Reinheit > 99 Gew.-%, > 99,9 Gew.-% oder sogar > 99,99 Gew.-% betragen kann, wird vorzugsweise zur Herstellung von Polyoxymethylen (POM), Polyoxymethylenderivaten wie Polyoxymethylendimethylether (POMDME) und Diaminodiphenylmethan (MDA) verwendet.

Die Erfindung wird nachfolgend mit Bezugnahme auf die Zeichnung näher erläutert.

Es zeigt:

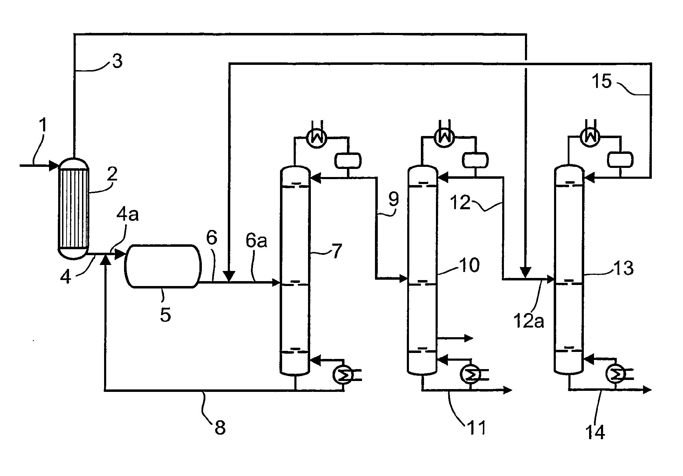

- Figur 1: beispielhaft eine Ausführungsform des erfindungsgemäßen Verfahrens.

Eine wässrige Formaldehydlösung **1** wird dem Verdampfer **2,** beispielsweise einem Dünnschichtverdampfer, Fallfilmverdampfer oder Wendelrohrverdampfer zugeführt. Als Brüdenabzugsstrom 3 (Strom IX) des Verdampfers wird eine an Formaldehyd abgereicherte wässrige Lösung, als Sumpfabzugsstrom **4** (Strom X) des Verdampfers eine formaldehydreiche wässrige Lösung erhalten. Diese wird mit dem formaldehydreichen Sumpfabzugsstrom **8** (Strom II) der ersten Destillationskolonne **7** zum Einspeisungsstrom **4a** (Strom Xa) vereinigt. Dieser wird dem Trioxan-Synthesereaktor **5,** der als Verdampfer, Rührbehälter, Fest- oder Fließbettreaktor ausgebildet ist, zugeführt. Das den Trioxan-Synthesereaktor verlassende Trioxan/Formaldehyd/Wasser-Gemisch **6** (Strom I) wird mit dem trioxanreichen Kopfabzugsstrom **15** (Strom VII) der dritten Destillationskolonne **13** zum Strom **6a** (Strom Ia) vereinigt. Der Strom **6a** wird der ersten Destillationskolonne **7** zugeführt und dort in einen Formaldehyd/Wasser-Strom **8** (Strom II) und einen Formaldehyd/Wasser/Trioxan-Strom **9** (Strom III) aufgetrennt. Dabei werden der Strom **8** als Sumpfabzugsstrom und der Strom **9** als Kopfabzugsstrom erhalten. Strom **8** wird mit Strom **4** vereinigt und in den Reaktor **5** zurückgeführt. Der Formaldehyd/Wasser/Trioxan-Strom **9** wird der Destillationskolonne **10** zugeführt und dort in einem Sumpfabzugsstrom **11** (Strom IV) aus im Wesentlichen reinem Trioxan und einen Kopfabzugsstrom **12** (Strom V), der überwiegend Trioxan und daneben Wasser und Formaldehyd enthält, aufgetrennt. Der Strom **12** wird mit dem formaldehydarmen wässrigen Brüdenabzugsstrom **3** (Strom IX) des Verdampfers **2** zum Strom **12a** (Strom Va) vereinigt. Dieser wird einer dritten Destillationskolonne **13** zugeführt und dort in einen Strom **14** (Strom VI), der im Wesentlichen aus Wasser besteht und den Rückführstrom **15** (Strom VII), der überwiegend Trioxan und daneben Wasser und Formaldehyd enthält, aufgetrennt.

### Beispiel

Bei der rechnerischen Simulation des in der Figur dargestellten Verfahrens wurden Stoffströme **6, 6a, 8, 9, 11, 12, 3, 12a, 14 und 15** der in den Tabellen angegebenen Zusammensetzungen erhalten. Dabei wurden folgende Parameter gewählt: Die erste Destillationsstufe wird bei einem Druck von 0,8 bar in einer Kolonne **7** mit 5 theoretischen Böden durchgeführt. Das Rücklaufverhältnis beträgt 1,25, die Kopftemperatur 85 °C und die Sumpftemperatur 97 °C. Der Zulauf **6a** befindet sich auf Höhe des 3. theoretischen Bodens. Die zweite Destillationsstufe wird bei einem Druck von 4,0 bar in einer Kolonne **10** mit 17 theoretischen Böden durchgeführt. Das Rücklaufverhältnis beträgt 0,1, die Kopftemperatur 131 °C und die Sumpftemperatur 167 °C. Der Zulauf **9** befindet sich auf Höhe des 10. theoretischen Bodens. Die dritte Destillationsstufe wird bei einem Druck von 2,5 bar in einer Kolonne **13** mit 25 theoretischen Böden durchgeführt. Das Rücklaufverhältnis beträgt 1,5, die Kopftemperatur 114 °C und die Sumpftemperatur 127 °C. Der Zulauf **12a** befindet sich auf Höhe des 20. theoretischen Bodens.

| Strom | 6 (I) | 6a | 8 | 9 | 11 | 12 | 3 | 12a | 14 | 15 |
|---|---|---|---|---|---|---|---|---|---|---|
| | | (Ia) | (II) | (III) | (IV) | (V) | (IX) | (Va) | (VI) | (VII) |
| Mengenstrom | 75,3 | 85,8 | 71,9 | 14,0 | 3,0 | 11,0 | 2,7 | 13,6 | 3,1 | 10,5 |
| [kg/h] | | | | | | | | | | |
| Formaldehyd | 70,5 | 64,1 | 74,5 | 10,5 | 0,0 | 13,4 | 15,0 | 13,7 | 0,07 | 17,8 |
| [Gew.-%] | | | | | | | | | | |
| Wasser | 25,5 | 24,4 | 25,5 | 19,1 | 0,001 | 24,3 | 85,0 | 36,1 | 99,92 | 17,2 |
| [Gew.-] | | | | | | | | | | |
| Trioxan | 4,0 | 11,5 | 0,01 | 70,4 | 99,999 | 62,3 | 0,0 | 50,2 | 0,01 | 65,1 |
| [Gew.-%] | | | | | | | | | | |

## Patentansprüche

1. Verfahren zur Abtrennung von Trioxan aus einem Einsatzstrom I aus Formaldehyd, Trioxan und Wasser, bei dem
a) ein Einsatzstrom **I,** der als Hauptkomponente Formaldehyd und als Nebenkomponenten Trioxan und Wasser enthält, bereitgestellt wird,
b) der Einsatzstrom **I** mit einem Rückführstrom VII, der als Hauptkomponente Trioxan und als Nebenkomponenten Formaldehyd und Wasser enthält, gemischt wird, wobei ein Einsatzstrom Ia, der als Hauptkomponente Formaldehyd und als Nebenkomponenten Trioxan und Wasser enthält, erhalten wird,
c) der Einsatzstrom Ia in einer ersten Destillationsstufe bei einem Druck von 0,1 bis 2,5 bar destilliert wird, wobei ein Strom II, der als Hauptkomponente Formaldehyd und als Nebenkomponente Wasser enthält, und ein Strom III, der als Hauptkomponente Trioxan und als Nebenkomponenten Wasser und Formaldehyd enthält, erhalten werden,
d) der Strom III, gegebenenfalls nach Abtrennung von Leichtsiedern aus dem Strom III in einer Leichtsieder-Abtrennstufe, in einer zweiten Destillationsstufe bei einem Druck von 0,2 bis 17,5 bar destilliert wird, wobei der Druck in der zweiten Destillationsstufe um 0,1 bis 15 bar höher als der Druck in der ersten Destillationsstufe ist, wobei ein Strom IV, der im wesentlichen aus Trioxan besteht, und ein Strom V, der als Hauptkomponente Trioxan und als Nebenkomponenten Wasser und Formaldehyd enthält, erhalten wird,
e) gegebenenfalls der Strom V mit einem Strom IX, der als Hauptkomponente Wasser enthält, gemischt wird, wobei ein Strom Va mit höherem Wassergehalt als Strom V, wobei der Strom Va als Hauptkomponenten Trioxan und als Nebenkomponenten Wasser und Formaldehyd enthält, erhalten wird,
f) der Strom V bzw. Va in einer dritten Destillationsstufe bei einem Druck von 1 bis 10 bar destilliert wird, wobei ein Strom VI, der im wesentlichen aus Wasser besteht, und der Rückführstrom VII, der als Hauptkomponente Trioxan und als Nebenkomponenten Wasser und Formaldehyd enthält, erhalten werden.

2. Verfahren nach Anspruch 1, **dadurch gekennzeichnet, dass** der Druck in der zweiten Destillationsstufe um 1,0 bis 10 bar höher als der Druck in der ersten Destillationsstufe ist.

3. Verfahren nach Anspruch 1 oder 2, **dadurch gekennzeichnet, dass** die erste Destillationsstufe bei einem Druck von 0,75 bis 1,25 bar durchgeführt wird.

4. Verfahren nach einem der Ansprüche 1 bis 3, **dadurch gekennzeichnet, dass** die dritte Destillationsstufe bei einem Druck von 2,5 bis 5 bar durchgeführt wird.

5. Verfahren nach einem der Ansprüche 1 bis 4, **dadurch gekennzeichnet, dass** die erste Destillationsstufe in einer ersten Destillationskolonne mit mindestens 2 theoretischen Böden, die zweite Destillationsstufe in einer zweiten Destillationskolonne mit mindestens 2 theoretischen Böden und die dritte Destillationsstufe in einer dritten Destillationskolonne mit mindestens 2 theoretischen Böden durchgeführt werden.

6. Verfahren nach Anspruch 5, **dadurch gekennzeichnet, dass** der Abtriebsteil der ersten Destillationskolonne 60 bis 90 % der Zahl der theoretischen Trennstufen dieser Kolonne aufweist.

7. Verfahren nach Anspruch 5 oder 6, **dadurch gekennzeichnet, dass** der Abtriebsteil der zweiten Destillationskolonne 50 bis 75 % der Zahl der theoretischen Trennstufen dieser Kolonne aufweist.

8. Verfahren nach einem der Ansprüche 5 bis 7, **dadurch gekennzeichnet, dass** der Abtriebsteil der dritten Destillationskolonne 70 bis 90 % der Zahl der theoretischen Trennstufen dieser Kolonne aufweist.

9. Verfahren nach einem der Ansprüche 1 bis 8, **dadurch gekennzeichnet, dass** zwischen der ersten und der zweiten Destillationsstufe eine Leichtsieder-Abtrennstufe durchgeführt wird, in der aus dem Strom III Leichtsieder, ausgewählt aus der Gruppe bestehend aus Methylformiat, Methylal, Dimethoxydimethylether und Methanol, abgetrennt werden.

10. Verfahren nach Anspruch 9, **dadurch gekennzeichnet, dass** die Leichtsiederabtrennung bei in einem Druck von 0,1 bis 5,0 bar in einer Destillationskolonne mit mindestens 2 theoretischen Stufen durchgeführt wird.

11. Verfahren nach einem der Ansprüche 1 bis 10, **gekennzeichnet durch** die nachstehende Zusammensetzung der Ströme I - VII:
Strom I: 60 bis 80 Gew.-% Formaldehyd, 15 bis 35 Gew.-% Wasser, 1 bis 15 Gew.-% Trioxan;
Strom Ia: 55 bis 75 Gew.-% Formaldehyd, 15 bis 35 Gew.-% Wasser, 3 bis 20 Gew.-% Trioxan;
Strom II: 65 bis 85 Gew.-% Formaldehyd, 15 bis 35 Gew.-% Wasser, 0 bis 1 Gew.-% Trioxan;
Strom III: 3 bis 20 Gew.-% Formaldehyd, 10 bis 30 Gew.-% Wasser, 60 bis 80 Gew.-% Trioxan;
Strom IV: 95 bis 100 Gew.-% Trioxan, 0 bis 5 Gew.-% Wasser und Nebenkomponenten;
Strom V: 5 bis 20 Gew.-% Formaldehyd, 15 bis 35 Gew.-% Wasser, 50 bis 75 Gew.-% Trioxan;
Strom Va: 5 bis 20 Gew.-% Formaldehyd, 25 bis 45 Gew.-% Wasser, 40 bis 65 Gew.-% Trioxan;
Strom VI: 0 bis 1 Gew.-% Formaldehyd, 99 bis 100 Gew.-% Wasser;
Strom VII: 5 bis 30 Gew.-% Formaldehyd, 5 bis 30 Gew.-% Wasser, 50 bis 80 Gew.-% Trioxan.
wobei die Ströme I, Ia, III, V, Va und VII noch bis zu 15 Gew.-% Leichtsieder, ausgewählt aus der Gruppe bestehend aus Methylformiat, Methylal, Dimethoxydimethylether und Methanol, enthalten können.

12. Verfahren zur Herstellung von Trioxan aus einer wässrigen Formaldehydlösung, bei dem ein Strom X aus einer wässrigen Formaldehydlösung einer Trioxan-Synthesestufe zugeführt und unter sauren Bedingungen umgesetzt wird, wobei der Strom I erhalten wird, und aus dem Strom I nach dem Verfahren gemäß einem der Ansprüche 1 bis 11 Trioxan abgetrennt wird.

13. Verfahren nach Anspruch 8 oder 9, **dadurch gekennzeichnet, dass** der Strom X aus einem Strom VIII aus einer wässrigen Formaldehydlösung niedrigerer Formaldehyd-Konzentration durch Aufkonzentrieren in einem Verdampfer erhalten wird.

14. Verfahren nach Anspruch 13, **dadurch gekennzeichnet, dass** der Strom IX der an Formaldehyd abgereicherte Brüdenabzugsstrom des Verdampfers ist.

## Claims

1. A process for removing trioxane from a use stream I of formaldehyde, trioxane and water, by
a) providing a use stream I which comprises formaldehyde as the main component and trioxane and water as the secondary components,
b) mixing the use stream I with a recycle stream VII which comprises trioxane as the main component and formaldehyde and water as the secondary components to obtain a feed stream la which comprises formaldehyde as the main component and trioxane and water as the secondary components,
c) distilling the use stream la in a first distillation stage at a pressure of from 0.1 to 2.5 bar to obtain a stream II which comprises formaldehyde as the main component and water as the secondary component, and a stream III which comprises trioxane as the main component and water and formaldehyde as the secondary components,
d) distilling the stream III, if appropriate after removing low boilers from the stream III in a low boiler removal stage, in a second distillation stage at a pressure of from 0.2 to 17.5 bar, the pressure in the second distillation stage being from 0.1 to 15 bar higher than the pressure in the first distillation stage, to obtain a stream IV which consists substantially of trioxane and a stream V which comprises trioxane as the main component and water and formaldehyde as the secondary components,
e) if appropriate mixing the stream V with a stream IX which comprises water as the main component to obtain a stream Va having a higher water content than the stream V, the stream Va comprising trioxane as the main component and water and formaldehyde as the secondary components,
f) distilling the stream V or Va in a third distillation stage at a pressure of from 1 to 10 bar to obtain a stream VI which consists substantially of water and the recycle stream VII which comprises trioxane as the main component and water and formaldehyde as the secondary components.

2. The process according to claim 1, wherein the pressure in the second distillation stage is from 1.0 to 10 bar higher than the pressure in the first distillation stage.

3. The process according to claim 1 or 2, wherein the first distillation stage is carried out at a pressure of from 0.75 to 1.25 bar.

4. The process according to any of claims 1 to 3, wherein the third distillation stage is carried out at a pressure of from 2.5 to 5 bar.

5. The process according to any of claims 1 to 4, wherein the first distillation stage is carried out in a first distillation column having at least two theoretical plates, the second distillation stage in a second distillation column having at least 2 theoretical plates and the third distillation stage in a third distillation column having at least two theoretical plates.

6. The process according to claim 5, wherein the stripping section of the first distillation column has from 60 to 90% of the number of theoretical plates of this column.

7. The process according to claim 5 or 6, wherein the stripping section of the second distillation column has from 50 to 75% of the number of theoretical plates of this column.

8. The process according to any of claims 5 to 7, wherein the stripping section of the third distillation column has from 70 to 90% of the number of theoretical plates of this column.

9. The process according to any of claims 1 to 8, wherein a low boiler removal stage is carried out between the first and the second distillation stage, in which low boilers selected from a group consisting of methyl formate, methylal, dimethoxydimethyl ether and methanol are removed from the stream III.

10. The process according to claim 9, wherein the low boiler removal is carried out at a pressure of from 0.1 to 5.0 bar in a distillation column having at least 2 theoretical plates.

11. The process according to any of claims 1 to 10, **characterized by** the following composition of streams I-VII:
stream I: from 60 to 80% by weight of formaldehyde, from 15 to 35% by weight of water, from 1 to 15% by weight of trioxane;
stream la: from 55 to 75% by weight of formaldehyde, 15 to 35% by weight of water, 3 to 20% by weight of trioxane;
stream II: from 65 to 85% by weight of formaldehyde, 15 to 35% by weight of water, 0 to 1 % by weight of trioxane;
stream III: from 3 to 20% by weight of formaldehyde, 10 to 30% by weight of water, 60 to 80% by weight of trioxane;
stream IV: from 95 to 100% by weight of trioxane, 0 to 5% by weight of water and secondary components;
stream V: from 5 to 20% by weight of formaldehyde, 15 to 35% by weight of water, 50 to 75% by weight of trioxane;
stream Va: from 5 to 20% by weight of formaldehyde, 25 to 45% by weight of water, 40 to 65% by weight of trioxane;
stream VI: from 0 to 1% by weight of formaldehyde, 99 to 100% by weight of water;
stream VII: from 5 to 30% by weight of formaldehyde, 5 to 30% by weight of water, 50 to 80% by weight of trioxane,
and the streams I, Ia, III, V, Va and VII may also comprise up to 15% by weight of low boilers selected from the group consisting of methyl formate, methylal, dimethoxydimethyl ether and methanol.

12. A process for preparing trioxane from an aqueous formaldehyde solution, by feeding a stream X of an aqueous formaldehyde of a trioxane synthesis stage and converting it under acidic conditions to obtain the stream I, and removing trioxane from the stream I by the process according to any of claims 1 to 11.

13. The process according to claim 8 or 9, wherein the stream X is obtained from a stream VIII from an aqueous formaldehyde solution of low formaldehyde concentration by concentrating in an evaporator.

14. The process according to claim 13, wherein the stream IX is the formaldehydedepleted vapor draw stream of the evaporator.

## Revendications

1. Procédé pour la séparation de trioxane d'un flux de départ I constitué par du formaldéhyde, du trioxane et de l'eau, dans lequel
a) on met à disposition un flux de départ I, qui contient comme composant principal du formaldéhyde et comme composants secondaires du trioxane et de l'eau,
b) on mélange le flux de départ I avec un flux recyclé VII, qui contient comme composant principal du trioxane et comme composants secondaires du formaldéhyde et de l'eau, avec obtention d'un flux de départ Ia, qui contient comme composant principal du formaldéhyde et comme composants secondaires du trioxane et de l'eau,
c) on distille le flux de départ Ia dans une première étape de distillation à une pression de 0,1 à 2,5 bars, avec obtention d'un flux II, qui contient comme composant principal du formaldéhyde et comme composants secondaires de l'eau, et d'un flux III, qui contient comme composant principal du trioxane et comme composants secondaires de l'eau et du formaldéhyde,
d) on distille le flux III, le cas échéant après séparation des substances à bas point d'ébullition du flux III dans une étape de séparation des substances à bas point d'ébullition, dans une deuxième étape de distillation à une pression de 0,2 à 17,5 bars, la pression dans la deuxième étape de distillation étant supérieure de 0,1 à 15 bars à la pression dans la première étape de distillation, avec obtention d'un flux IV, qui est essentiellement constitué de trioxane, et d'un flux V, qui contient comme composant principal du trioxane et comme composants secondaires de l'eau et du formaldéhyde,
e) on mélange le cas échéant le flux V avec un flux IX, qui contient comme composant principal de l'eau, avec obtention d'un flux Va présentant une teneur en eau plus élevée que le flux V, le flux Va contenant comme composant principal du trioxane et comme composants secondaires de l'eau et du formaldéhyde,
f) on distille le flux V ou Va dans une troisième étape de distillation à une pression de 1 à 10 bars, avec obtention d'un flux VI, qui est essentiellement constitué d'eau et du flux de recyclage VII, qui contient comme composant principal du trioxane et comme composants secondaires de l'eau et du formaldéhyde.

2. Procédé selon la revendication 1, **caractérisé en ce que** la pression dans la deuxième étape de distillation est supérieure de 1,0 à 10 bars à la pression dans la première étape de distillation.

3. Procédé selon la revendication 1 ou 2, **caractérisé en ce que** la première étape de distillation est réalisée à une pression à 0,75 jusqu'à 1,25 bar.

4. Procédé selon l'une quelconque des revendications 1 à 3, **caractérisé en ce que** la troisième étape de distillation est réalisée à une pression à 2,5 jusqu'à 5 bars.

5. Procédé selon l'une quelconque des revendications 1 à 4, **caractérisé en ce que** la première étape de distillation est réalisée dans une première colonne de distillation présentant au moins 2 plateaux théoriques, la deuxième étape de distillation dans une deuxième colonne de distillation présentant au moins 2 plateaux théoriques et la troisième étape de distillation dans une troisième colonne de distillation présentant au moins 2 plateaux théoriques.

6. Procédé selon la revendication 5, **caractérisé en ce que** la zone de rectification de la première colonne de distillation présente 60 à 90% du nombre d'étages de séparation théoriques de cette colonne.

7. Procédé selon la revendication 5 ou 6, **caractérisé en ce que** la zone de rectification de la deuxième colonne de distillation présente 50 à 75% du nombre d'étages de séparation théoriques de cette colonne.

8. Procédé selon l'une quelconque des revendications 5 à 7, **caractérisé en ce que** la zone de rectification de la troisième colonne de distillation présente 70 à 90% du nombre d'étages de séparation théoriques de cette colonne.

9. Procédé selon l'une quelconque des revendications 1 à 8, **caractérisé en ce qu'**on réalise, entre la première et la deuxième étape de distillation, une étape de séparation des substances à bas point d'ébullition, dans laquelle on élimine du flux III des substances de bas point d'ébullition choisies dans le groupe constitué par le formiate de méthyle, le méthylal, le diméthoxydiméthyléther et le méthanol.

10. Procédé selon la revendication 9, **caractérisé en ce que** la séparation des substances à bas point d'ébullition est réalisée à une pression à 0,1 jusqu'à 5,0 bars dans une colonne de distillation présentant au moins 2 étages théoriques.

11. Procédé selon l'une quelconque des revendications 1 à 10, **caractérisé par** la composition suivante des flux I - VII :
Flux I : 60 à 80% en poids de formaldéhyde, 15 à 35% en poids d'eau, 1 à 15% en poids de trioxane ;
Flux Ia : 55 à 75% en poids de formaldéhyde, 15 à 35% en poids d'eau, 3 à 20% en poids de trioxane ;
Flux II : 65 à 85% en poids de formaldéhyde, 15 à 35% en poids d'eau, 0 à 1% en poids de trioxane ;
Flux III : 3 à 20% en poids de formaldéhyde, 10 à 30% en poids d'eau, 60 à 80% en poids de trioxane ;
Flux IV : 95 à 100% en poids de trioxane, 0 à 5% en poids d'eau et des composants secondaires ;
Flux V : 5 à 20% en poids de formaldéhyde, 15 à 35% en poids d'eau, 50 à 75% en poids de trioxane ;
Flux Va : 5 à 20% en poids de formaldéhyde, 25 à 45% en poids d'eau, 40 à 65% en poids de trioxane ;
Flux VI : 0 à 1% en poids de formaldéhyde, 99 à 100% en poids d'eau ;
Flux VII : 5 à 30% en poids de formaldéhyde, 5 à 30% en poids d'eau, 50 à 80% en poids de trioxane ;
les flux I, Ia, III, V, Va et VII pouvant encore contenir jusqu'à 15% en poids de substances à bas point d'ébullition, choisies dans le groupe formé par le formiate de méthyle, le méthylal, le diméthoxydiméthyléther et le méthanol.

12. Procédé pour la préparation de trioxane à partir d'une solution aqueuse de formaldéhyde, dans lequel on alimente un flux X d'une solution aqueuse de formaldéhyde d'une étape de synthèse de trioxane et on le transforme dans des conditions acides, avec obtention du flux I et on sépare du flux I selon le procédé selon l'une quelconque des revendications 1 à 11 du trioxane.

13. Procédé selon la revendication 8 ou 9, **caractérisé en ce que** le flux X est obtenu à partir d'un flux VIII d'une solution aqueuse de formaldéhyde présentant une faible concentration en formaldéhyde par concentration dans un évaporateur.

14. Procédé selon la revendication 13, **caractérisé en ce que** le flux IX est le flux d'élimination des condensats de buées appauvri en formaldéhyde de l'évaporateur.
